# EUROPEAN PATENT SPECIFICATION

(11) **EP 2 158 929 B1**
(45) Date of publication and mention of the grant of the patent: **16.03.2011**
(21) Application number: 09010166.8
(22) Date of filing: 06.08.2009
(51) Int. Cl.: A61M 5/158

(54) **Subcutaneous infusion device**
Subkutanes Infusionsgerät
Dispositif de perfusion sous-cutanée

(30) Priority: 29.08.2008 JP 2008221962; 29.08.2008 JP 2008221963; 29.08.2008 JP 2008221964
(43) Date of publication of application: 03.03.2010
(73) Proprietor: NIPRO CORPORATION, Kita-ku, Osaka-shi, Osaka-fu, 531-8510 (JP)
(72) Inventor: Ishikura, Kohzo, Osaka-shi Osaka, 531-8510 (JP); Ishihara, Naoko, Osaka-shi Osaka, 531-8510 (JP); Yamaguchi, Takeshi, Osaka-shi Osaka, 531-8510 (JP)
(74) Representative: Bertsch, Florian Oliver

(56) References cited:
- EP-A- 0 956 879
- WO-A-98/58693
- WO-A-2006/020851
- US-A1- 2004 158 207
- US-A1- 2005 107 743

## Description

This nonprovisional application is based on Japanese Patent Applications Nos. 2008-221962, 2008-221963, and 2008-221964 all filed on August 29, 2008 with the Japan Patent Office.

### BACKGROUND OF THE INVENTION

### Field of the Invention

The present invention relates to a subcutaneous infusion device, and particularly relates to a subcutaneous infusion device for infusing a medical solution into the body of a user.

### Description of the Background Art

A subcutaneous infusion device used for Continuous Subcutaneous Insulin Infusion (CSII), for example, has been known conventionally. Its specific examples include the ones described in, for example, the specification of U.S. Patent Application Laying-Open No. 2006/0129090 (Patent Document 1), the specification of U.S. Patent Application Laying-Open No. 2003/0216686 (Patent Document 2), the specification of U.S. Patent No. 6056718 (Patent Document 3), and others.

Other specific examples include the ones described in, for example, the specification of U.S. Patent No. 6926694 (Patent Document 4), the specification of U.S. Patent Application Laying-Open No. 2002/0173769 (Patent Document 5), the specification of U.S. Patent Application Laying-Open No. 2006/0276760 (Patent Document 6), and others.

Further specific examples include the ones described in, for example, Japanese Patent Laying-Open No. 2004-305467 (Patent Document 7), the specification of International Publication WO98/58693 (Patent Document 8), and others.

The above-described continuous subcutaneous insulin infusion is identified as medical treatment for constantly infusing insulin by subcutaneously indwelling an indwelling needle, so as to accurately control blood-sugar levels. However, in the daily life of a patient (e.g. bathing, exercising, and the like), a fluid infusion line of the subcutaneous infusion device becomes obstructive and tends to decrease convenience in the patient's life. Therefore, in recent years, there has been provided a mechanism that makes a fluid infusion line detachable/attachable, and allows only the minimum necessary parts required for infusing a medical solution to be secured on the skin.

Usually, the detachable/attachable mechanism means a coupling mechanism and a separating mechanism between a section secured on the skin (indwelling member) when the fluid infusion line is separated and a section extending from a pump to the indwelling member (coupling member). These two mechanisms enable the indwelling member and the coupling member to be coupled/separated as many times as needed.

To operate the two mechanisms described above, a delicate operation is generally required. However, some of the patients who undergo treatment of continuous subcutaneous insulin infusion have peripheral neuropathy and others, and hence suffer from diminished sensation in the fingers, find difficulties in performing a delicate operation, and tend to find it difficult to smoothly manipulate the coupling mechanism and the separating mechanism.

Patent Document 1 shows a subcutaneous infusion device that has a coupling member attached to an indwelling member in a detachable/attachable manner. However, in the subcutaneous infusion device described in Patent Document 1, it is necessary to rotate the coupling member, which has been inserted into the indwelling member from a prescribed direction, with respect to the indwelling member. As such, the subcutaneous infusion device according to Patent Document 1 requires a delicate operation in manipulating the coupling and separating mechanisms.

Patent Document 2 shows a subcutaneous infusion device that utilizes a hinge as the coupling and separating mechanisms between the indwelling member and the coupling member. However, when the indwelling member is actually secured on the skin, it is often secured at a section that is difficult to check by visual inspection (the lower abdomen, the buttocks, or the like), so that in actual use, it is difficult to insert the coupling member into the hinge portion.

Patent Document 3 shows an example of a subcutaneous infusion device in which a coupling member is attached to an indwelling member by being slid along an extending direction of the indwelling member. A "coupling" manipulation becomes easier in the subcutaneous infusion device according to Patent Document 3 when compared with the subcutaneous infusion devices according to Patent Documents 1 and 2. However, a "separating" manipulation is still difficult to perform.

Further, in the subcutaneous infusion devices described in Patent Documents 4 and 5, a puncture portion of an indwelling member is located at a recessed site, and hence the direction along which the coupling member is attached is limited, resulting in that attachment of the coupling member is sometimes difficult.

Further, in the subcutaneous infusion device described in Patent Document 6, a direction along which the coupling member is attached is limited to a direction running from upward toward a surface of the body, and hence attachment of the coupling member is sometimes difficult.

Further, from a viewpoint totally different from the foregoing, it is important, in a subcutaneous infusion device in which an indwelling member and a coupling member are repeatedly coupled and separated, to ensure sterility and prevent a user from being exposed to a medical solution when the indwelling member is separated from the coupling member.

In the subcutaneous infusion devices described in Patent Documents 7 and 8, when the coupling member is separated from the indwelling member, a puncture needle provided at the coupling member is exposed to the outside. This is not preferable from the above-described viewpoints of ensuring sterility and preventing a user from being exposed to a medical solution. WO 2006/020851 discloses a subcutaneous infusion device according to the preamble of claim 1.

### SUMMARY OF THE INVENTION

An object of the present invention is to provide a subcutaneous infusion device that enables easy detachment/attachment of a coupling member from/to an indwelling member.

A subcutaneous infusion device according to the present invention is, in one aspect, a subcutaneous infusion device for infusing a medial solution into a body of a user, and includes: an indwelling member having a base portion fixable to a surface of the body of the user, and a medical solution passage reaching an inside of the body of the user when the base portion is fixed to the surface of the body of the user; and a coupling member attached to the indwelling member in a detachable/attachable manner by being moved in a sliding manner along an extending direction of the base portion. The coupling member includes a main body portion, a needle-like medical solution supply portion provided at the main body portion and communicated with the medical solution passage when the coupling member is attached to the indwelling member, a lid portion provided turnably with respect to the main body portion, a lock mechanism providing locking of the lid portion to the main body portion, and a biasing portion capable of biasing the lid portion in a turning direction, when the locking is being released, by biasing force generated when the lid portion is locked to the main body portion.

According to the above-described configuration, the coupling member can be attached to the indwelling member by being moved in a sliding manner along the extending direction of the base portion of the indwelling member, so that the coupling member can easily be attached to the indwelling member. Further, the biasing portion provided at the coupling member can bias the lid portion in the turning direction, so that the coupling member can easily be detached from the indwelling member. As such, there is provided a subcutaneous infusion device that enables easy detachment/attachment of the coupling member from/to the indwelling member.

Preferably in the above-described subcutaneous infusion device, the coupling member has a hook portion which suppresses a sliding movement of the coupling member along the extending direction of the base portion when the lid portion is locked to the main body portion.

Preferably in the above-described subcutaneous infusion device, the main body portion of the coupling member is formed into an approximately U shape, and the locking of the lid portion to the main body portion is released by increasing a spacing between opposite ends of the approximately U shape.

According to the above-described configuration, the locking of the lid portion to the main body portion can be released by pinching a prescribed position of the coupling member from opposite sides, so that there is no need to lift the lid portion in the upward direction (the direction away from the surface of the body of the user) to release the locking. Without such a configuration, it is necessary to press the indwelling member with one hand and detach the coupling member with the other hand when the coupling member is to be detached, so as to prevent the indwelling member from falling off from the body of the patient as the lid portion is lifted in the upward direction. In contrast, with the above-described configuration, there is no need to press the indwelling member when the coupling member is detached, so that the coupling member can be detached with one hand. Accordingly, the coupling member can more easily be detached from the indwelling member.

Preferably in the above-described subcutaneous infusion device, the indwelling member has a protruding portion which protrudes outward from the body of the user with respect to the base portion, and the main body portion of the coupling member has an opening which receives the protruding portion.

Preferably in the above-described subcutaneous infusion device, the indwelling member has a rotation-suppressing portion which suppresses rotation of the coupling member about an axis orthogonal to the extending direction of the base portion when the coupling member is attached to the indwelling member.

Preferably in the above-described subcutaneous infusion device, the indwelling member has a flange portion, the coupling member has a rail portion which engages with the flange portion, and the flange portion and the rail portion suppress a movement of the coupling member along a direction orthogonal to the extending direction of the base portion when the coupling member is attached to the indwelling member.

Preferably in the above-described subcutaneous infusion device, the coupling member includes a cover member fixed to the main body portion, formed to cover the medical solution supply portion when the coupling member is detached from the indwelling member, and formed to receive compression force from the indwelling member and be compressed in an axial direction to thereby allow the medical solution supply portion to penetrate the cover member and allow the medical solution supply portion and the medical solution passage to be communicated with each other, when the coupling member is attached to the indwelling member.

According to the above-described configuration, by providing the cover member formed to cover the medical solution supply portion when the coupling member is detached from the indwelling member, it is possible to prevent the puncture needle provided at the coupling member from being exposed to the outside when the coupling member is detached from the indwelling member. As a result, it is possible to provide a subcutaneous infusion device capable of reliably ensuring sterility and preventing the user from being exposed to a medical solution when the indwelling member is separated from the coupling member.

Preferably in the above-described subcutaneous infusion device, the indwelling member has a protruding portion which protrudes outward from the body of the user with respect to the base portion, and the cover member abuts on the protruding portion when the coupling member is attached to the indwelling member.

According to the above-described configuration, it is possible to compress and deform the cover member which has abutted on the protruding portion, to thereby allow the medical solution supply portion to easily penetrate the cover member.

Preferably in the above-described subcutaneous infusion device, the cover member has a self-closing property which enables a hole portion formed by penetration of the medical solution supply portion to be closed when the coupling member is detached from the indwelling member.

According to the above-described configuration, the self-closing property of the cover member enables the hole portion formed by penetration of the medical solution supply portion to be closed, and hence it is much easier to ensure sterility and prevent the user from being exposed to a medical solution when the indwelling member is separated from the coupling member.

Preferably in the above-described subcutaneous infusion device, a concave portion is provided at a side surface of the cover member. It is thereby possible to easily compress and deform the cover member, and hence allow the medical solution supply portion to easily penetrate the cover member.

Preferably in the above-described subcutaneous infusion device, the indwelling member further has a partition portion which defines the medical solution passage and a fastening portion which fastens the partition portion onto the base portion. The partition portion includes a puncture portion into which the medical solution supply portion is inserted. The puncture portion is formed to protrude more upward than the fastening portion protrudes in a state where the medical solution supply portion is detached from the indwelling member. It is noted that the "upward" in the specification of the present application means a direction apart from the surface of the body of the user.

According to the above-described configuration, the puncture portion protrudes more upward than the fastening portion does in a state where the medical solution supply portion is detached from the indwelling member, and hence a sterilizing operation prior to attachment of the medical solution supply portion to the indwelling member can easily be carried out. Further, the medical solution supply portion can be attached from upward as well as from a side. As a result, there is provided a subcutaneous infusion device that enables easy hygienic control and easy attachment of the medical solution supply portion.

Preferably in the above-described subcutaneous infusion device, the coupling member further includes a press portion which presses the partition portion when the coupling member is attached to the indwelling member.

According to the above-described configuration, even if a relatively soft material is used for the partition portion, the press portion compresses the partition portion so that the periphery of the hole portion pierced with the needle-like medical solution supply portion can reliably be closed, after the coupling member is attached to the indwelling member. Therefore, it is possible to facilitate both of reduction in puncture resistance and improvement in airtightness of the puncture portion.

Preferably, the above-described subcutaneous infusion device further includes a cover member fixed to the coupling member and formed to cover the medical solution supply portion when the coupling member is detached from the indwelling member. The cover member is formed to abut on the indwelling member and receive compression force to thereby be compressed in an axial direction, and allow the medical solution supply portion to penetrate the cover member and the puncture portion and allow the medical solution supply portion to be communicated with the medical solution passage, when the coupling member is attached to the indwelling member.

According to the above-described configuration, by providing the cover member formed to cover the medical solution supply portion when the coupling member is detached from the indwelling member, hygienic control of the medical solution supply portion becomes easier. As a result, there is provided a subcutaneous infusion device that enables much easier hygienic control.

Preferably in the above-described subcutaneous infusion device, the puncture portion of the indwelling member has a self-closing property which enables a hole portion formed by penetration of the medical solution supply portion to be closed when the coupling member is detached from the indwelling member.

According to the above-described configuration, the self-closing property of the puncture portion enables the hole portion formed by penetration of the medical solution supply portion to be closed, and hence hygienic control of the medical solution passage inside the partition becomes easy when the coupling member is detached from the indwelling member.

The above-described subcutaneous infusion device is preferably used for continuous subcutaneous insulin infusion.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a perspective view that shows a state prior to attachment of a coupling member of a subcutaneous infusion device according to a first embodiment of the present invention to an indwelling member.
Fig. 2 is a perspective view that shows a state where the coupling member of the subcutaneous infusion device according to the first embodiment of the present invention is attached to the indwelling member.
Fig. 3 is a cross-sectional view taken along III-III in Fig. 1.
Fig. 4 is a cross-sectional side view that shows an intermediate state between the state shown in Fig. 3 and a state shown in Fig. 5.
Fig. 5 is a cross-sectional view taken along V-V in Fig. 2.
Fig. 6 is a perspective view that shows an indwelling member included in the subcutaneous infusion device shown in Figs. 1-5.
Fig. 7 is a cross-sectional view taken along VII-VII in Fig. 6.
Fig. 8 is a top view of a coupling member included in the subcutaneous infusion device shown in Figs. 1-5.
Fig. 9 is a bottom view of the coupling member included in the subcutaneous infusion device shown in Figs. 1-5.
Fig. 10 is a side view of the coupling member included in the subcutaneous infusion device shown in Figs. 1-5.
Fig. 11 is a cross-sectional side view of the coupling member included in the subcutaneous infusion device shown in Figs. 1-5.
Figs. 12A and 12B are drawings that show a lock mechanism for a main body portion and a lid portion of the coupling member shown in Figs. 8-11 Figs. 13A-13C are drawings for describing a shape of a needle cover.
Fig. 14 is a perspective view that shows a state prior to attachment of a coupling member of a subcutaneous infusion device according to a second embodiment of the present invention to an indwelling member
Fig. 15 is a perspective view that shows a state where the coupling member of the subcutaneous infusion device according to the second embodiment of the present invention is attached to the indwelling member.
Fig. 16 is a drawing that shows a state seen from the direction of an arrow XVI in Fig. 14.
Fig. 17 is a drawing that shows a state seen from the direction of an arrow XVII in Fig. 15.
Fig. 18 is a cross-sectional side view that shows a state where a coupling member of a subcutaneous infusion device according to a third embodiment of the present invention is attached to an indwelling member.
Fig. 19 is a perspective view that shows a state where a coupling member of a subcutaneous infusion device according to a fourth embodiment of the present invention is attached to an indwelling member.
Fig. 20 is a cross-sectional view taken along XX-XX in Fig. 19.
Fig. 21 is a bottom view of a coupling member included in a subcutaneous infusion device according to a fifth embodiment of the present invention, and shows only the coupling member when it is attached to an indwelling member.
Fig. 22 is a cross-sectional side view of the coupling member included in the subcutaneous infusion device according to the fifth embodiment of the present invention, and shows only the coupling member when it is attached to the indwelling member.
Fig. 23 is a (first) cross-sectional side view that shows a state where the coupling member of the subcutaneous infusion device according to the fifth embodiment of the present invention is attached to the indwelling member.
Fig. 24 is a (second) cross-sectional side view that shows a state where the coupling member of the subcutaneous infusion device according to the fifth embodiment of the present invention is attached to the indwelling member.
Fig. 25 is a perspective view that shows a state prior to attachment of a coupling member of a subcutaneous infusion device according to a sixth embodiment of the present invention to an indwelling member.
Fig. 26 is a perspective view that shows a state where the coupling member of the subcutaneous infusion device according to the sixth embodiment of the present invention is attached to the indwelling member.
Fig. 27 is a cross-sectional view of the indwelling member shown in Figs. 25 and 26.

### DESCRIPTION OF THE PREFERRED EMBODIMENTS

Embodiments of the present invention will hereinafter be described. It is noted that the same or corresponding portions are provided with the same reference characters, and the description thereof may not be repeated.

It is also noted that if the number, amount, and others are referred to in the embodiments described below, the scope of the present invention is not necessarily limited to the number, amount, and others unless otherwise specified. Further, each component in the embodiments below is not necessarily essential to the present invention unless otherwise specified. Further, if a plurality of embodiments are provided in the following, it is intended from the beginning to combine as appropriate the configurations of the embodiments unless otherwise specified.

### (First Embodiment)

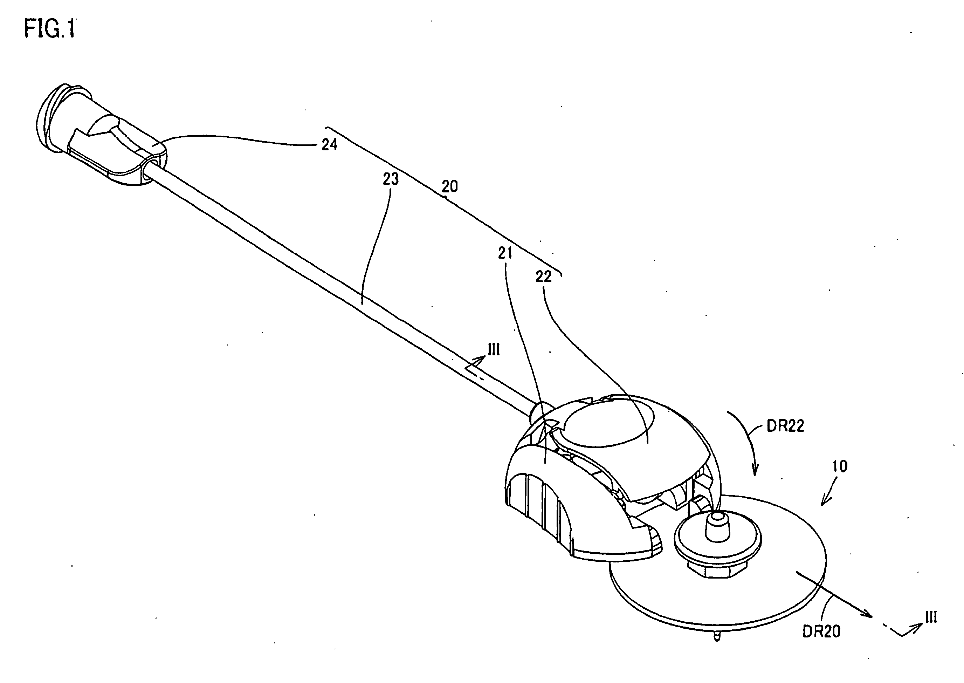

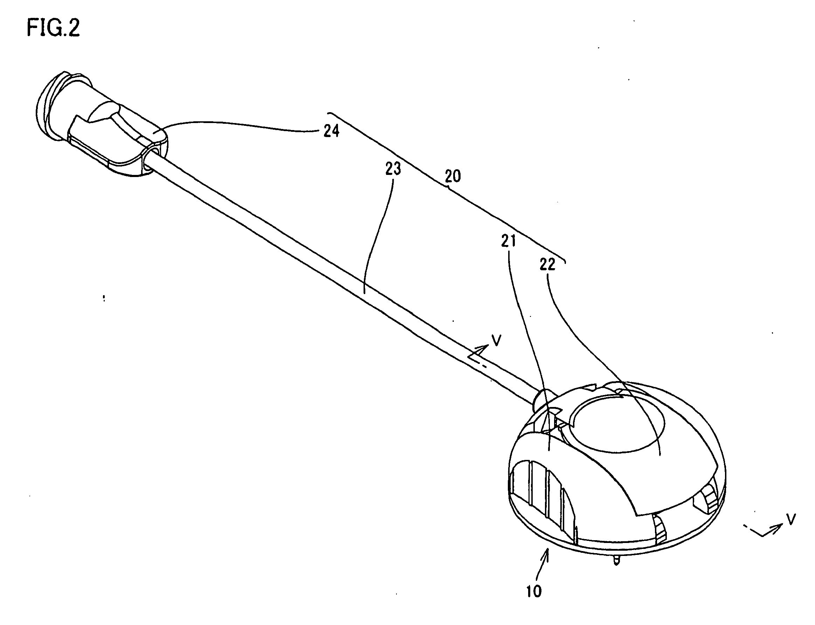

Fig. 1 is a perspective view that shows a state prior to attachment of a coupling member of a subcutaneous infusion device according to a first embodiment to an indwelling member. Fig. 2 is a perspective view that shows a state where the coupling member of the subcutaneous infusion device is attached to the indwelling member.

With reference to Figs. 1 and 2, a subcutaneous infusion device according to the present embodiment is used for continuous subcutaneous insulin infusion (CSII), and configured to include an indwelling member 10 and a coupling member 20. Indwelling member 10 is a member for being secured at a surface of the body of a patient when continuous subcutaneous insulin infusion is carried out. Coupling member 20 is a member for being detached/attached as needed from/to indwelling member 10. In other words, when insulin is subcutaneously infused, coupling member 20 is coupled to indwelling member 10, and when infusion of insulin is suspended (e.g. during bathing and the like), coupling member 20 is separated from indwelling member 10.

Coupling member 20 includes a main body portion 21, a lid portion 22 turnably provided with respect to main body portion 21, a fluid infusion line 23, and a connector 24. By sliding main body portion 21 and lid portion 22 of coupling member 20 in the direction of an arrow DR20 (the direction along the surface of the body of the patient), and then turning lid portion 22 of coupling member 20 in the direction of an arrow DR22, coupling member 20 is attached to indwelling member 10 fixed to the surface of the body of the patient. Connector 24 is connected to a pump (not shown) supplying a medical solution. The medical solution supplied from the pump (not shown) to fluid infusion line 23 through connector 24 is guided into the body of the patient through a medical solution passage in main body portion 21 and a medical solution passage in indwelling member 10.

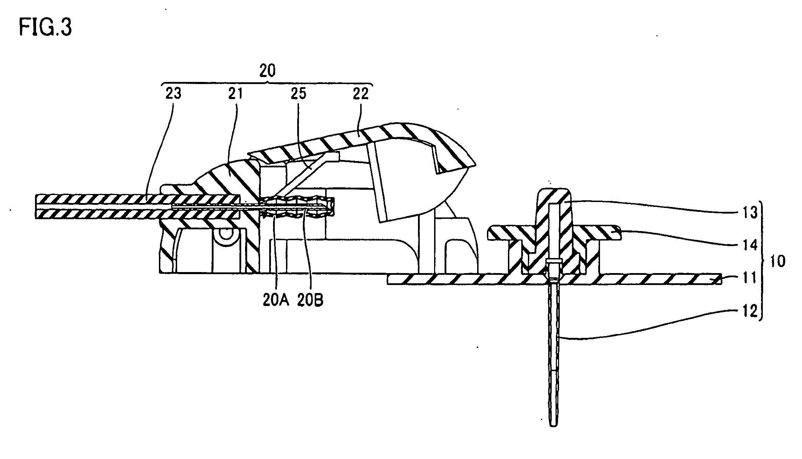

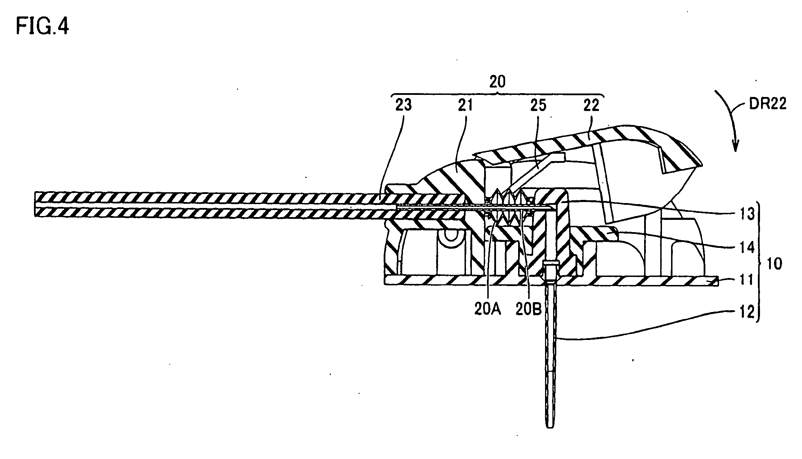

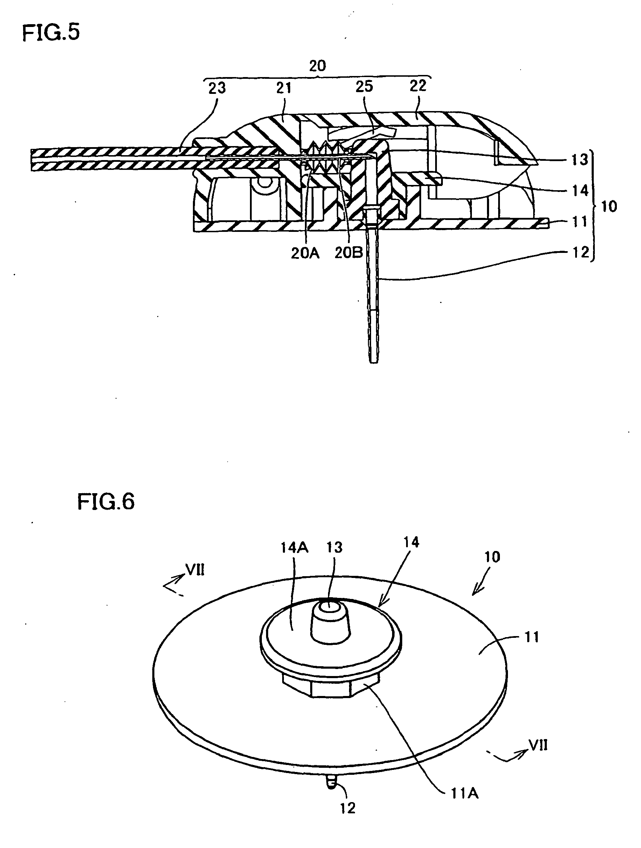

Figs. 3 and 5 are a cross-sectional view taken along III-III in Fig. 1 and a cross-sectional view taken along V-V in Fig. 2, respectively. Fig. 4 is a cross-sectional side view that shows an intermediate state between the state shown in Fig. 3 and the state shown in Fig. 5.

With reference to Figs. 3-5, indwelling member 10 includes a base portion 11, a cannula 12, a partition portion 13, and a fastening portion 14. Indwelling member 10 is fixed to a prescribed position on the surface of the body of the patient (e.g. the lower abdomen or the buttocks), with the use of, for example, an apparatus provided with a needle for subcutaneous introduction and referred to as an inserter. However, indwelling member 10 may also be fixed by utilizing a needle for subcutaneous introduction, without using an inserter. When indwelling member 10 is fixed to the surface of the body of the patient, cannula 12 reaches an inside of the body of the patient. At a lower surface of base portion 11, an adhesion layer (not shown in Figs. 3-5) made of a nonwoven fabric, for example, is provided, and the adhesion layer causes indwelling member 10 to be fixed to the surface of the body of the patient. Partition portion 13 and fastening portion 14 are provided on base portion 11. Fastening portion 14 fastens partition portion 13 onto base portion 11 Partition portion 13 is provided such that a part of partition portion 13 protrudes from fastening portion 14.

Coupling member 20 further includes a bottomed tube-like needle cover 20A and a hollow needle 20B, in addition to main body portion 21, lid portion 22, fluid infusion line 23, and connector 24 described above. One end side of needle cover 20A is fixed to main body portion 21, and the other end side thereof is made as a free end. To needle 20B, a medical solution is supplied through fluid infusion line 23.

As shown in Fig. 3, needle 20B is accommodated in needle cover 20A before coupling member 20 is attached to indwelling member 10. When coupling member 20 is moved in a sliding manner from this state, needle cover 20A abuts on partition portion 13 and is compressed and deformed, and needle 20B penetrates needle cover 20A and partition portion 13, as shown in Fig. 4. Subsequently, by turning lid portion 22 in the direction of arrow DR22, the state shown in Fig. 5 is reached. Needle 20B and cannula 12 are thereby communicated with each other, so that the medical solution from the pump is guided into the body of the patient. It is noted that needle cover 20A is formed in a bellows-like manner for easy compression and deformation.

It is noted that coupling member 20 further includes an elastic member 25. An action of elastic member 25 will later be described.

Cannula 12 of indwelling member 10 preferably has flexibility and strength to a certain degree so as to adapt, while being inserted into the body of the patient, to movements in his/her daily life. Cannula 12 is formed of, for example, a resin such as fluoroplastic, polyethylene, polypropylene, or polyurethane.

Partition portion 13 of indwelling member 10 and needle cover 20A of coupling member 20 are required to allow needle 20B to penetrate therethrough when coupling member 20 is attached to indwelling member 10, and are required to close a hole made by needle 20B when coupling member 20 is detached from indwelling member 10. Therefore, partition portion 13 and needle cover 20A preferably have a self-closing property to a certain degree. Partition portion 13 and needle cover 20A are formed of, for example, an elastic material such as natural rubber, synthetic polyisoprene rubber, butyl rubber, chloroprene rubber, silicon rubber, urethane rubber, styrene-butadiene rubber, ethylene-propylene rubber, acrylic rubber, fluororubber, or thermoplastic elastomer.

Needle 20B of coupling member 20 is configured with metal such as stainless steel, or rigid plastic such as polycarbonate, polypropylene, or ABS resin. Needle 20B can thereby penetrate needle cover 20A with ease. Further, portions of indwelling member 10 and coupling member 20 other than cannula 12, partition portion 13, needle cover 20A, and needle 20B are formed of, for example, polyolefin such as polyethylene, polypropylene, polybutadiene, or ethylene-vinyl acetate copolymer, polyester such as polyvinyl chloride, polyurethane, polystyrene, polymethyl methacrylate, polycarbonate, polyamide, polyethylene terephthalate, or polybutylene terephthalate, or a resin material such as acrylic resin, ABS resin, ionomer, polyacetal, polyphenylene sulfide, or polyether ether ketone.

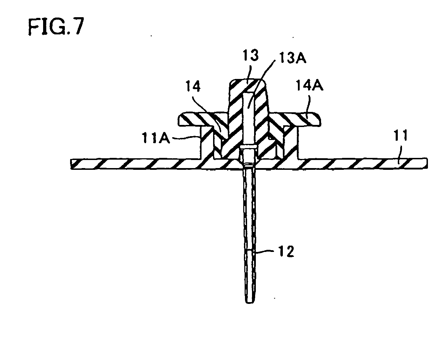

Fig. 6 is a perspective view that shows indwelling member 10 Fig. 7 is a cross-sectional view taken along VII-VII in Fig. 6.

With reference to Figs. 6 and 7, indwelling member 10 includes a guide portion 11A that protrudes outward from the body of the user with respect to base portion 11. Guide portion 11A guides a sliding movement of coupling member 20, which is detached from/attached to indwelling member 10. Guide portion 11A has a shape of an even-numbered-sided regular polygon (a regular octagon in the present embodiment) If guide portion 1 1A has the shape of a regular octagon, each of four pairs of opposite sides functions as guide surfaces that guide a sliding movement of coupling member 20, and hence a sliding movement of coupling member 20 can be guided along four different directions. By adapting to a sliding movement in a plurality of directions as such, it becomes possible to select as appropriate from the direction from which coupling member 20 is attached to indwelling member 10, so that coupling member 20 can easily be attached to/detached from indwelling member 10. Further, guide portion 11A formed to have a polygonal shape also functions as a "rotation-suppressing portion" that suppresses rotation of coupling member 20 about an axis orthogonal to the extending direction of base portion 11 (about an axis in the up-and-down direction in Figs. 6 and 7) when coupling member 20 is attached to indwelling member 10.

As shown in Figs. 6 and 7, base portion 11 has a thin disc shape. Partition portion 13 has an internal space 13A, and internal space 13A and cannula 12 are communicated with each other. Fastening portion 14 has a flange portion 14A. Flange portion 14A also has a disc shape as base portion 11 does. Although flange portion 14A is provided to protrude radially outward with respect to guide portion 1 1A and partition portion 13, it has a diameter smaller than a diameter of base portion 11. A part of partition portion 13 protrudes more upward than flange portion 14A does. When coupling member 20 is attached to indwelling member 10, needle 20B is inserted into the portion of partition portion 13, which portion protrudes more upward than flange portion 14A does.

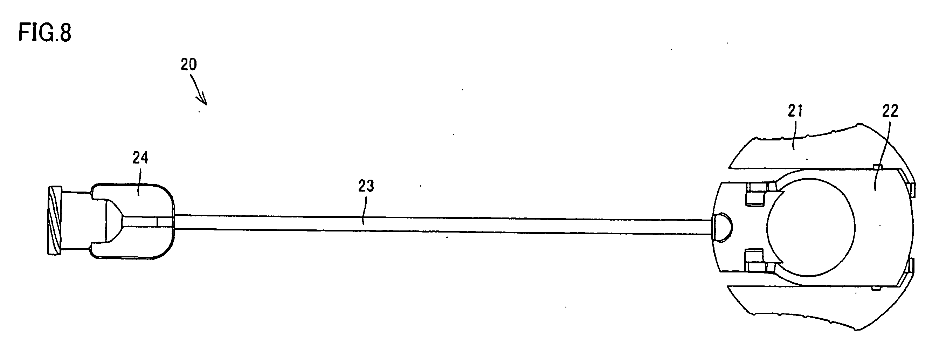

Figs. 8-11 are a top view, a bottom view, a side view, and a cross-sectional side view of coupling member 20, respectively.

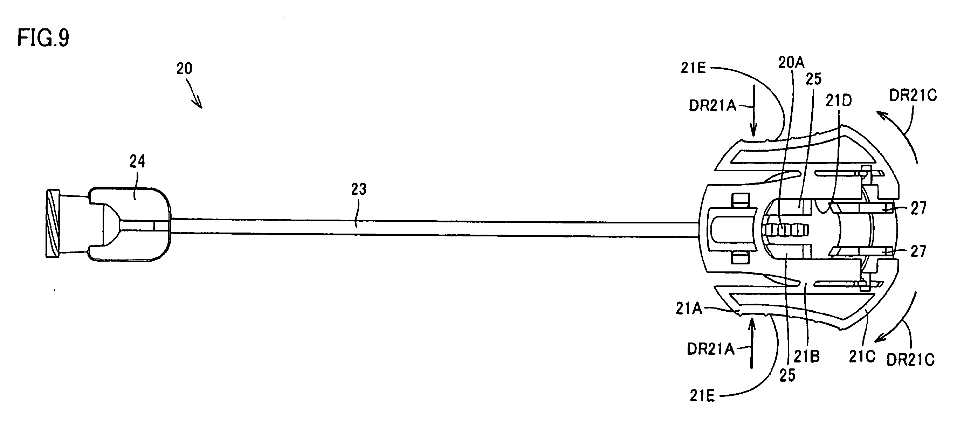

With reference to Figs. 8-11, main body portion 21 of coupling member 20 is formed into an approximately U shape. More specifically, main body portion 21 has a rear end portion 21 A, a fulcrum portion 21B, a front end portion 21 C, and an opening 21D. Guide portion 11A of indwelling member 10 is received in opening 21D. As shown in Fig. 9, by pressing rear end portion 21A in the direction of an arrow DR21 A (i.e. clamping main body portion 21 from the opposite sides), front end portion 2 1 C is deformed in the direction of an arrow DR21C with fulcrum portion 21B serving as a fulcrum. A spacing between the opposite ends of coupling member 20, which is formed into an approximately U shape, is thereby increased, so that locking of lid portion 22 to main body portion 21 is released. It is noted that a concave portion 21E that has a shape easily conforming to the shape of the fingers is formed at a side surface of main body portion 21. It becomes thereby easier to pinch main body portion 21 with two fingers.

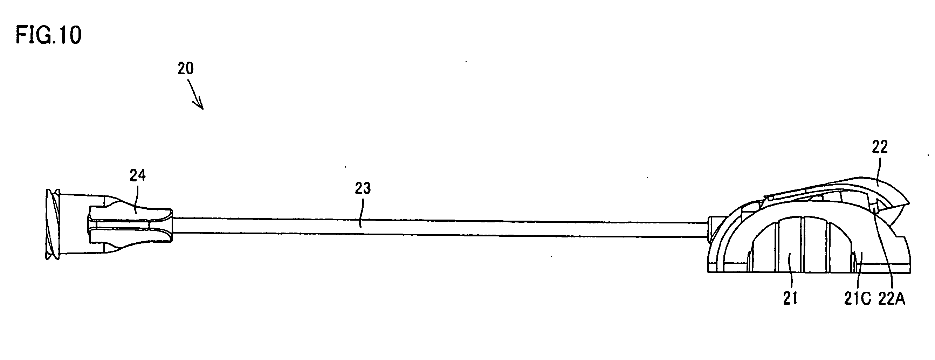

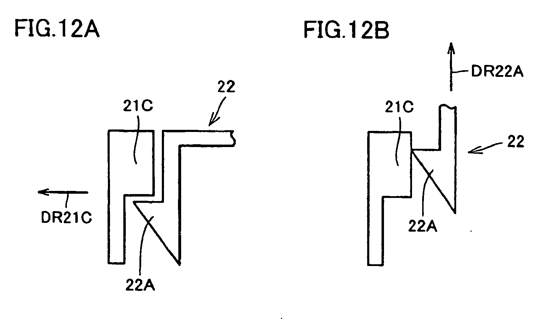

It is noted that the lock mechanism for main body portion 21 and lid portion 22 is configured with front end portion 21 C of main body portion 21 and an engagement portion 22A engaging with front end portion 21 C, as shown in Fig. 10 In other words, by deforming front end portion 21 C in the direction of arrow DR21C (see Fig. 12A), engagement of front end portion 21 C and engagement portion 22A is released, so that lid portion 22 turns in an opening direction (in the direction of arrow DR22A) (see Fig. 12B).

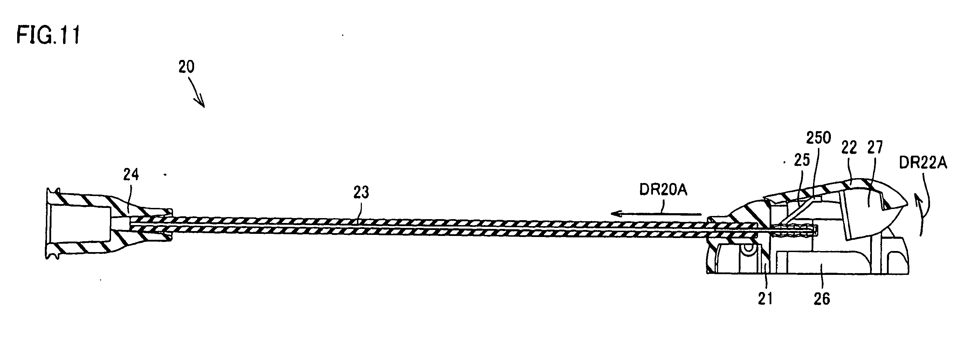

As shown in Fig. 11, coupling member 20 has a rail portion 26 that engages with flange portion 14A formed at indwelling member 10. Flange portion 14A of indwelling member 10 and rail portion 26 restrain coupling member 20 in a direction orthogonal to the extending direction of base portion 11, when coupling member 20 is attached to indwelling member 10.

Further, lid portion 22 has a hook portion 27. Hook portion 27 abuts on indwelling member 10 when lid portion 22 is locked to main body portion 21. Coupling member 20 is thereby restrained in the extending direction of base portion 11, when coupling member 20 is attached to indwelling member 10.

As described above, coupling member 20 is prevented from falling off from indwelling member 10. Coupling member 20 is further provided with elastic member 25 as described above. Elastic member 25 is configured with, for example, a leaf spring formed in a strip-like manner. One end side (base side) of elastic member 25 is fixed to main body portion 21, and the other end side (tip side) of elastic member 25 is made as a free end. As shown in Fig. 11, a tip portion 250 of elastic member 25 abuts on lid portion 22 in a state where lid portion 22 is opened. Therefore, in a state where lid portion 22 is closed (a state where lid portion 22 is locked to main body portion 21), biasing force is generated at elastic member 25. It is noted that although two elastic members 25 are provided in a bilaterally symmetric manner in the present embodiment as shown in Fig. 9, one or more than two elastic member(s) 25 may be provided. Further, as elastic member 25, a known elastic member such as a coil spring may also be used instead of a leaf spring.

When lid portion 22 is locked to main body portion 21, elastic member 25 is deformed between main body portion 21 and lid portion 22, and between indwelling member 10 and coupling member 20 (the state shown in Fig. 5). When coupling member 20 is to be detached from indwelling member 10, rear end portion 21 A of coupling member 20 is pressed in the direction of arrow DR21A to increase a spacing between the opposite ends of coupling member 20, so that the locking of lid portion 22 and main body portion 21 is released as described above. Since elastic member 25 is deformed as described above, biasing force (resilience) of elastic member 25 biases lid portion 22 in its turning direction (the direction of arrow DR22A in Fig. 11). Consequently, coupling member 20 can easily be detached from indwelling member 10. Further, by modifying as appropriate the position and the like for providing elastic member 25, elastic member 25 is capable of biasing lid portion 22 in the turning direction as well as biasing coupling member 20 in the sliding direction (in the direction of an arrow DR20A in Fig. 11) As a result, coupling member 20 can more easily be detached from indwelling member 10.

As described above, in the subcutaneous infusion device according to the present embodiment, coupling member 20 can be attached to indwelling member 10 by being moved in a sliding manner along the extending direction of base portion 11 of indwelling member 10, so that coupling member 20 can easily be attached to indwelling member 10. Further, elastic member 25 provided at coupling member 20 can bias lid portion 22 in the turning direction (the direction of arrow DR22A), so that coupling member 20 can easily be detached from indwelling member 10. As such, according to the present embodiment, there is provided a subcutaneous infusion device that enables easy detachment/attachment of coupling member 20 from/to indwelling member 10.

Further, by increasing a spacing between the opposite ends of the approximately U shape of main body portion 21 of coupling member 20 to thereby release the locking of lid portion 22 to main body portion 21, the locking of lid portion 22 to main body portion 21 can be released by pinching a prescribed position of coupling member 20 from the opposite sides. Therefore, there is no need to lift lid portion 22 in the upward direction so as to release the locking. In the conventional subcutaneous infusion device that does not have such a configuration, it is necessary to press indwelling member 10 with one hand and detach coupling member 20 with the other hand when coupling member 20 is to be detached, so as prevent indwelling member 10 from falling off from the body of the patient as lid portion 22 is lifted in the upward direction. In contrast, in the subcutaneous infusion device according to the present embodiment, there is no need to press indwelling member 10 when coupling member 20 is to be detached, and it becomes possible to detach coupling member 20 with one hand. Therefore, coupling member 20 can more easily be detached from indwelling member 10.

Further, in the subcutaneous infusion device according to the present embodiment, by providing needle cover 20A formed to cover needle 20B when coupling member 20 is detached from indwelling member 10, needle 20B provided at coupling member 20 can be prevented from being exposed to the outside when coupling member 20 is separated from indwelling member 10. Consequently, it is possible to provide a subcutaneous infusion device capable of reliably ensuring sterility and preventing a user from being exposed to a medical solution when indwelling member 10 is separated from coupling member 20.

Further, a self-closing property of needle cover 20A enables the hole portion formed by penetration of needle 20B to be closed, so that it is much easier to ensure sterility and prevent a user from being exposed to a medical solution when indwelling member 10 is separated from coupling member 20.

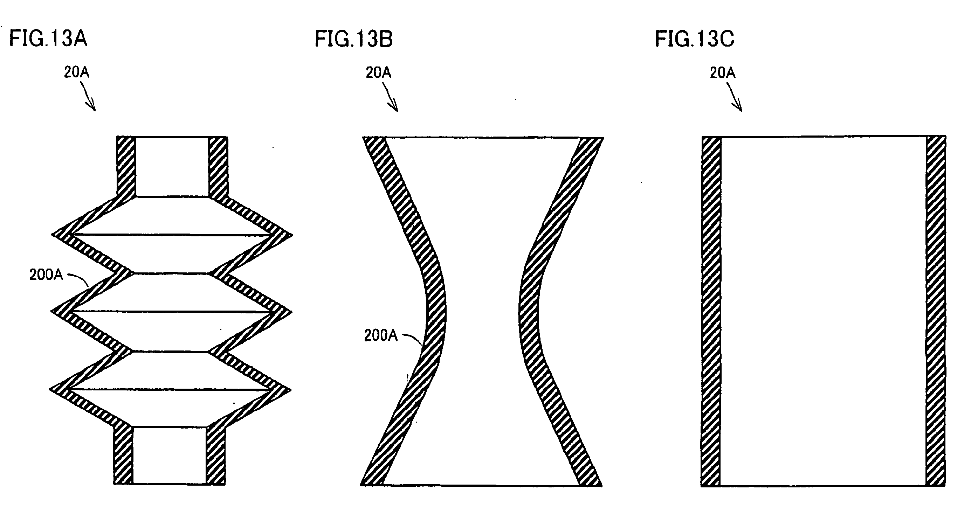

Here, a modification of the shape of needle cover 20A will be described. Although needle cover 20A formed in a bellows-like manner has been shown in the present embodiment as shown in Fig. 13A, the shape of needle cover 20A may be the one having a smaller diameter at the middle portion in the axial direction as shown in Fig. 13B, or alternatively, may be the one having a simple cylindrical shape as shown in Fig. 13C. It is noted that when a concave portion 200A is formed at a side surface of needle cover 20A as shown in Figs. 13A and 13B, needle cover 20A can more easily be compressed and deformed in the axial direction, so that puncture resistance is reduced, and needle 20B can easily penetrate needle cover 20A.

Further in the subcutaneous infusion device according to the present embodiment, puncture portion 13B pierced with needle 20B of coupling member 20 is exposed to the outside in a state where coupling member 20 is detached from indwelling member 10. Therefore, a sterilizing operation prior to attachment of coupling member 20 to indwelling member 10 can easily be carried out, and consequently, a subcutaneous infusion device that enables easy hygienic control is provided. Further, puncture portion 13 protrudes more upward than fastening portion 14 does, and hence needle 20B can be inserted from a side of partition portion 13 as described above. In other words, by allowing puncture portion 13 to protrude more upward than fastening portion 14 does, needle 20B can be inserted from upward as in the conventional subcutaneous infusion device (Patent Document 3), and can also be inserted from a side. As a result, there is provided a subcutaneous infusion device that enables easy attachment of coupling member 20.

Further, in the subcutaneous infusion device according to the present embodiment, a self-closing property of puncture portion 13B enables the hole portion formed at puncture portion 13B by penetration of needle 20B of coupling member 20 to be closed. Therefore, hygienic control of internal space 13A inside partition portion 13 when coupling member 20 is detached from indwelling member 10 becomes easy.

The foregoing is summarized as follows. Specifically, in one aspect, the subcutaneous infusion device according to the present embodiment is a subcutaneous infusion device for infusing a medical solution into a body of a user, and includes: indwelling member 10 having base portion 11 fixable to a surface of the body of the user, and cannula 12 serving as a "medical solution passage" that reaches an inside of the body of the user when base portion 11 is fixed to the surface of the body of the user; and coupling member 20 attached to indwelling member 10 in a detachable/attachable manner by being moved in a sliding manner along an extending direction of base portion 11. Coupling member 20 includes main body portion 21, needle 20B serving as a "medical solution supply portion" that is provided at main body portion 21 and communicated with cannula 12 when coupling member 20 is attached to indwelling member 10, lid portion 22 provided turnably with respect to main body portion 21, engagement portion 22A serving as a "lock mechanism" that provides locking of lid portion 22 to main body portion 21, and elastic member 25 serving as a "biasing portion" capable of biasing lid portion 22 in a turning direction, when locking is being released, by biasing force generated when lid portion 22 is locked to main body portion 21.

Further, in the subcutaneous infusion device according to the present embodiment, partition portion 13 of indwelling member 10 includes puncture portion 13B into which needle 20B serving as a "medical solution supply portion" is inserted. Puncture portion 13B is formed to protrude more upward than fastening portion 14 does in a state where needle 20B is detached from indwelling member 10. Needle 20B is provided at coupling member 20. Coupling member 20 is attached to indwelling member 10 in a detachable/attachable manner by being moved in a sliding manner along the extending direction of base portion 11. Needle 20B is communicated with cannula 12 when coupling member 20 is attached to indwelling member 10.

Further, in an aspect different from the above-described one, the subcutaneous infusion device according to the present embodiment is a subcutaneous infusion device for infusing a medical solution into a body of a user, and includes: indwelling member 10 having base portion 11 fixable to a surface of the body of the user, and cannula 12 serving as a "medical solution passage" that reaches an inside of the body of the user when the base portion 11 is fixed to the surface of the body of the user; and coupling member 20 attached to indwelling member 10 in a detachable/attachable manner by being moved in a sliding manner along the extending direction of base portion 11. Coupling member 20 includes main body portion 21, needle 20B serving as a "medical solution supply portion" that is provided at main body portion 21 and communicated with cannula 12 when coupling member 20 is attached to indwelling member 10, and needle cover 20A serving as a "cover member" that is fixed to main body portion 21, formed to cover needle 20B when coupling member 20 is detached from indwelling member 10, and formed to receive compression force from indwelling member 10 and be compressed in the axial direction to thereby allow needle 20B to penetrate needle cover 20A and allow needle 20B and cannula 12 to be communicated with each other, when coupling member 20 is attached to indwelling member 10.

More specifically, indwelling member 10 has partition portion 13 serving as a "protruding portion" protruding outward from the body of the user with respect to base portion 11, and needle cover 20A abuts on partition portion 13 when coupling member 20 is attached to indwelling member 10.

It is noted that, although the subcutaneous infusion device used for continuous subcutaneous insulin infusion has been described in the present embodiment, the above-described subcutaneous infusion device can also be applied to an application for supplying another medical solution to an inside of the body (e.g. pain treatment and others).

### (Second Embodiment)

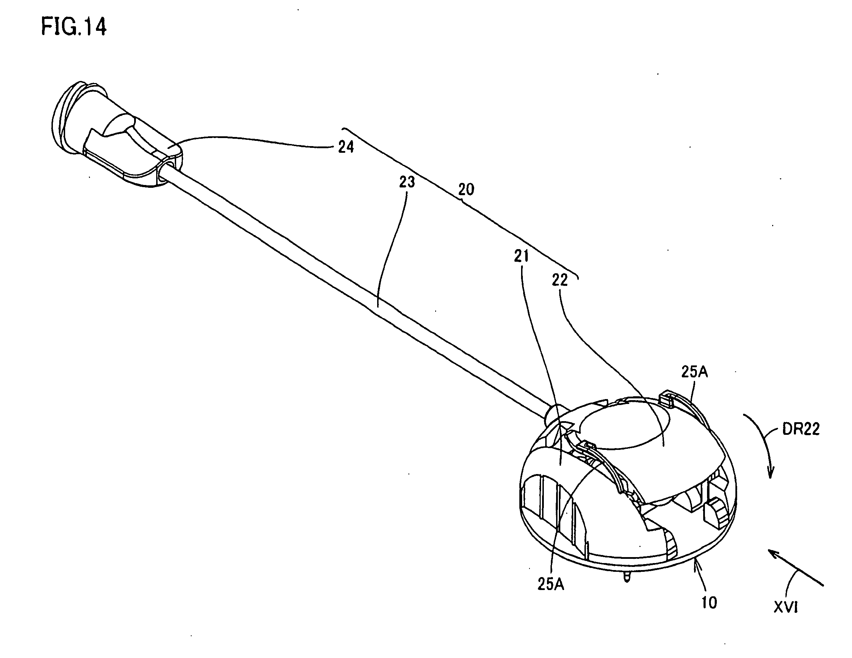

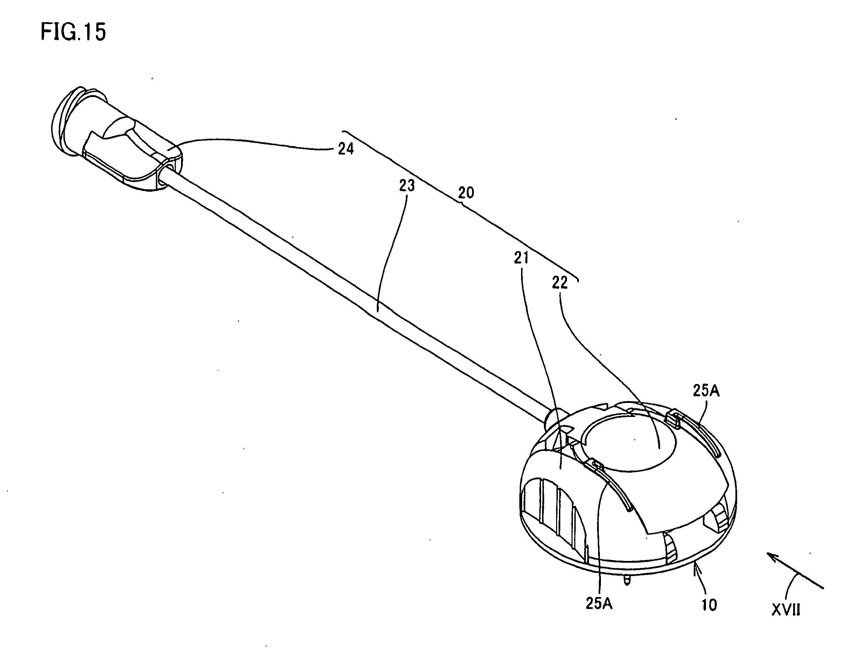

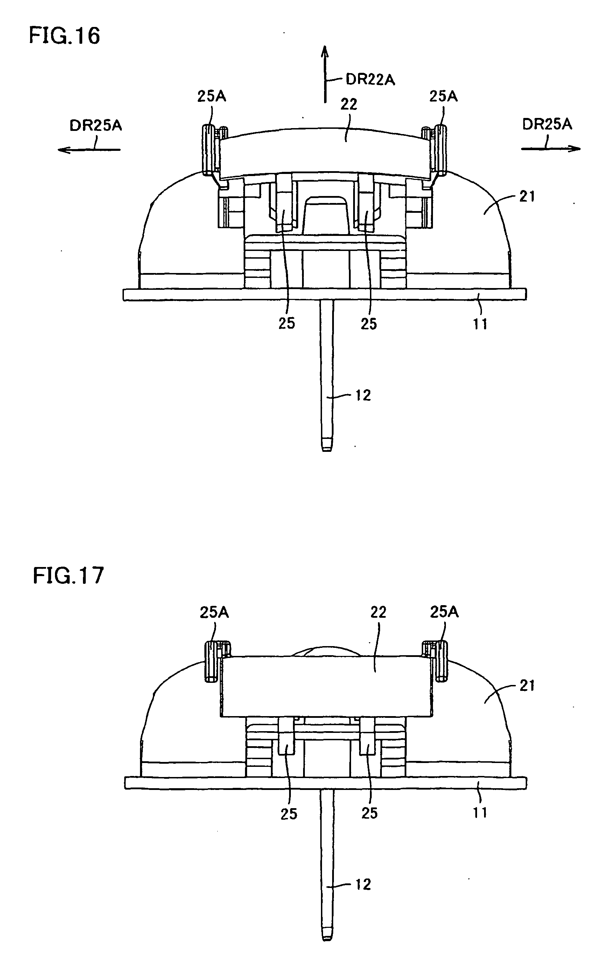

Figs. 14 and 15 are a perspective view that shows a state prior to attachment of coupling member 20 of a subcutaneous infusion device according to a second embodiment of the present invention to indwelling member 10, and a perspective view that shows a state where coupling member 20 is attached to indwelling member 10, respectively. Further, Figs. 16 and 17 are a drawing that shows a state seen from the direction of an arrow XVI in Fig. 14, and a drawing that shows a state seen from the direction of an arrow XVII in Fig. 15, respectively.

With reference to Figs. 14-17, a subcutaneous infusion device according to the present embodiment is a modification of the subcutaneous infusion device according to the first embodiment, characterized in that an elastic member 25A is provided in addition to elastic member 25, as a biasing member in removing coupling member 20. As shown in Figs. 14-17, elastic member 25A is provided on each of opposite sides of lid portion 22. One end side (base side) of elastic member 25A is fixed to lid portion 22, while the other end side (tip side) of elastic member 25A is made as a free end. In a state where locking of lid portion 22 and main body portion 21 is released as shown in Fig. 14, the tip of elastic member 25A somewhat projects outward (to an arrow DR25A side) from an edge of a fitting portion of main body portion 21. When lid portion 22 is locked to main body portion 21, elastic member 25A is deformed to be oriented somewhat inward as shown in Fig. 17. Therefore, when the locking of lid portion 22 and main body portion 21 is released again, lid portion 22 easily turns in the direction of arrow DR22A by biasing force of elastic member 25A in the direction of arrow DR25A. As such, in the subcutaneous infusion device according to the present embodiment, locking of lid portion 22 can more easily be released when compared with the subcutaneous infusion device according to the first embodiment.

### (Third Embodiment)

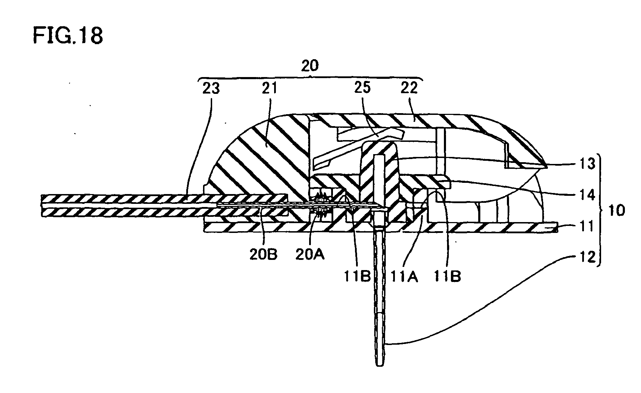

Figs. 18 is a cross-sectional side view that shows a state where a coupling member of a subcutaneous infusion device according to a third embodiment is attached to an indwelling member. With reference to Fig. 18, a subcutaneous infusion device according to the present embodiment is a modification of the subcutaneous infusion devices according to the first and second embodiments, characterized to have a structure in which needle 20B is inserted through an insertion hole 11B provided at guide portion 11A of indwelling member 10. In other words, in the subcutaneous infusion device according to the present embodiment, guide portion 11A configures a "protruding portion", and when coupling member 20 is attached to indwelling member 10, needle cover 20A abuts on guide portion 11A and is compressed and deformed.

In the subcutaneous infusion device according to the present embodiment, locking of lid portion 22 can easily be released as in the first and second embodiments. Further, it is possible to reliably ensure sterility and prevent a user from being exposed to a medical solution when indwelling member 10 is separated from coupling member 20.

### (Fourth Embodiment)

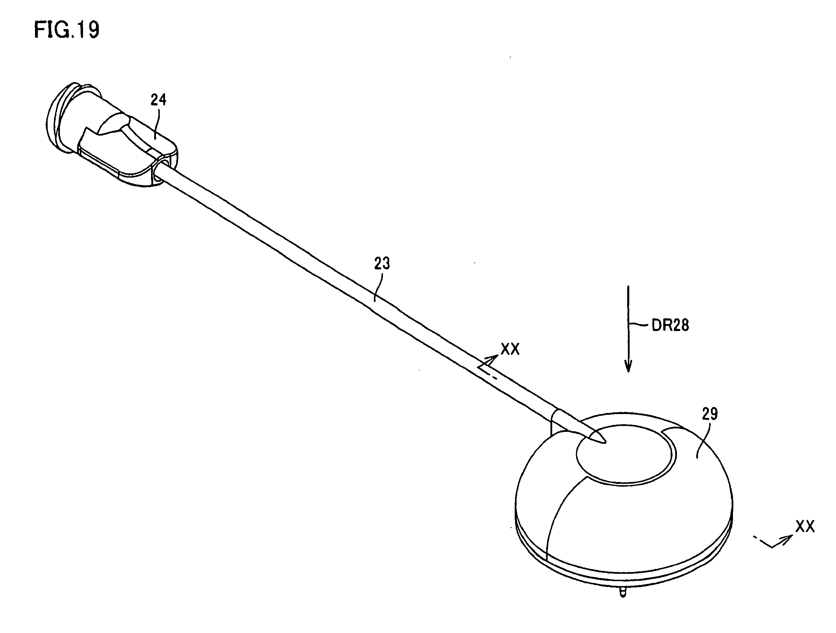

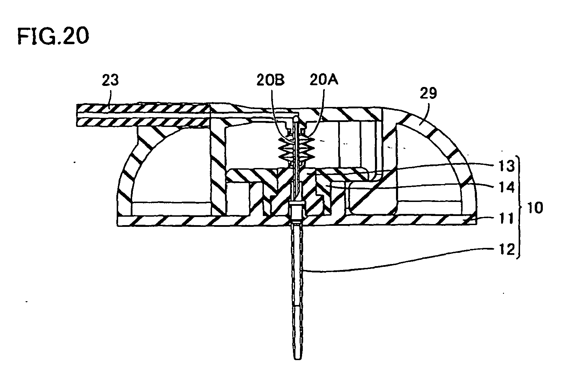

Fig. 19 is a perspective view that shows a state where a coupling member of a subcutaneous infusion device according to a fourth embodiment is attached to an indwelling member. Fig. 20 is a cross-sectional view taken along XX-XX in Fig. 19. With reference to Figs. 19 and 20, a subcutaneous infusion device according to the present embodiment is a modification of the subcutaneous infusion devices according to the first to third embodiments, characterized to have a structure in which coupling member 20 is attached to indwelling member 10 from an upper side (a side apart from the surface of the body of the user). In other words, in the subcutaneous infusion device according to the present embodiment, as shown in Fig. 20, needle cover 20A and needle 20B are provided in the up-and-down direction, and when main body 29 is attached to indwelling member 10 from an upper side, needle cover 20A abuts on the upper surface of partition portion 13 and is compressed and deformed.

In the subcutaneous infusion device according to the present embodiment, locking of lid portion 22 can easily be released as in the first to third embodiments. Further, it is possible to reliably ensure sterility and prevent a user from being exposed to a medical solution when indwelling member 10 is separated from coupling member 20.

### (Fifth Embodiment)

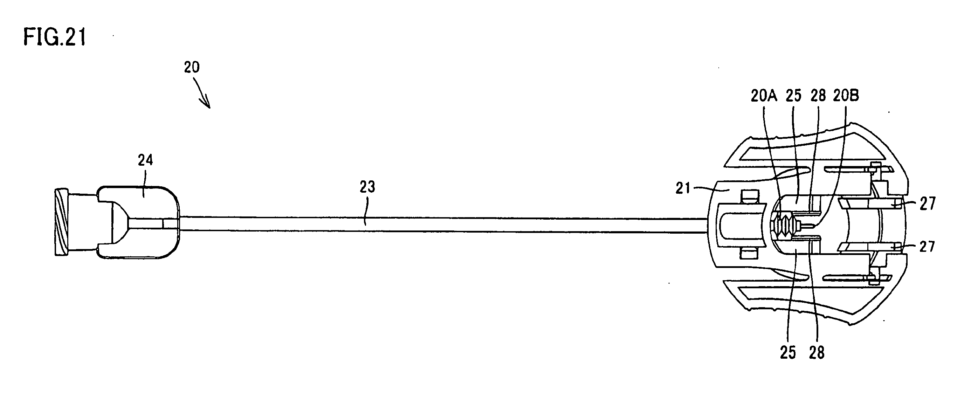

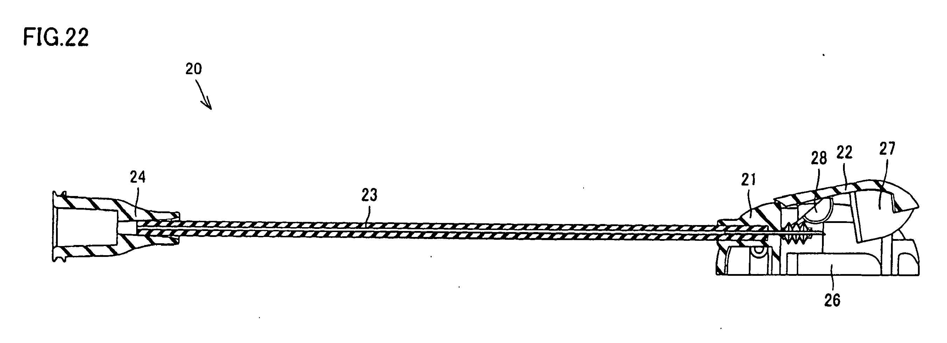

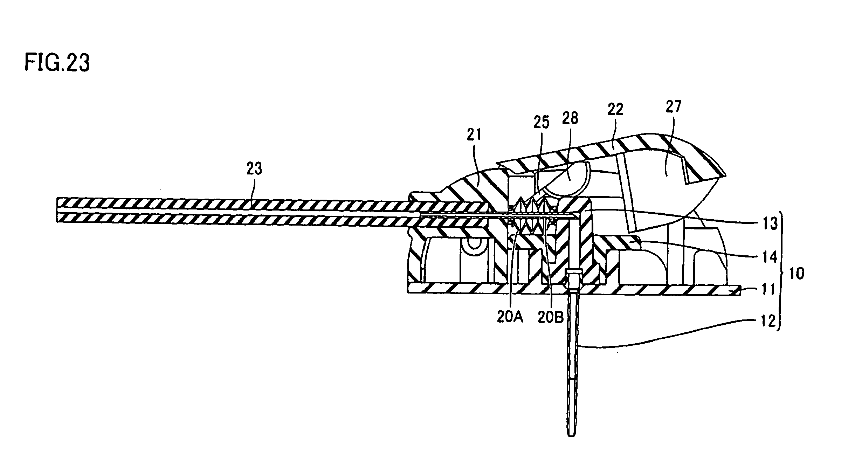

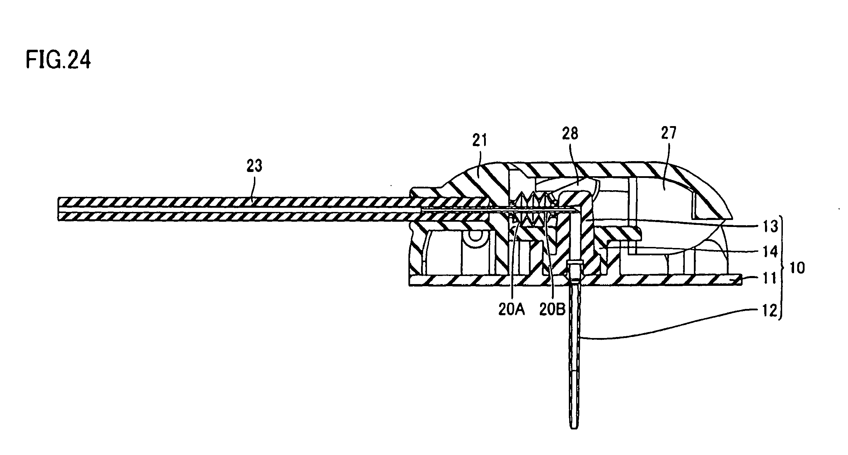

Figs. 21 and 22 are a bottom view and a cross-sectional side view of a coupling member included in a subcutaneous infusion device according to a fifth embodiment, respectively, and show only the coupling member when it is attached to the indwelling member. Figs. 23 and 24 are cross-sectional side views that show a state where the coupling member of the subcutaneous infusion device is attached to the indwelling member (Fig. 23 shows a state before lid portion 22 is closed, and Fig. 24 shows a state after lid portion 22 is closed).

With reference to Figs. 23-24, a subcutaneous infusion device according to the present embodiment is a modification of the subcutaneous infusion devices according to the first to fourth embodiments, characterized in that a press portion 28 turning as lid portion 22 turns at coupling member 20 and pressing partition portion 13, is further provided.

As shown in Figs. 21 and 22, press portion 28 is fixed to elastic member 25.

An upper end of press portion 28 abuts on lid portion 22. Further, as shown in Fig. 14, a lower end of press portion 28 abuts on partition portion 13 in a state where lid portion 22 is opened. As shown in Fig. 15, when lid portion 22 is turned from this state and closed, press portion 28 presses partition portion 13 toward the surface of the body of the user at its opposite end portions in its width direction (the direction running from the back to the front on the plane of Figs. 23 and 24). By doing so, after coupling member 20 is attached to indwelling member 10, press portion 28 compresses partition portion 13, so that a periphery of the hole portion pierced with needle 20B can reliably be closed. Accordingly, a relatively soft material can be used for partition portion 13, so that it is possible to facilitate both of reduction in puncture resistance of puncture portion 13B, and improvement in airtightness of puncture portion 13B after being punctured.

Although the example in which partition portion 13 is pressed toward the surface of the body of the user has been described in the present embodiment, the direction along which partition portion 13 is pressed is not limited thereto. For example, if needle 20B is inserted into partition portion 13 from an upper side of partition portion 13, partition portion 13 is pressed in a direction approximately parallel with the surface of the body of the user in a typical example, after needle 20B is inserted.

### (Sixth Embodiment)

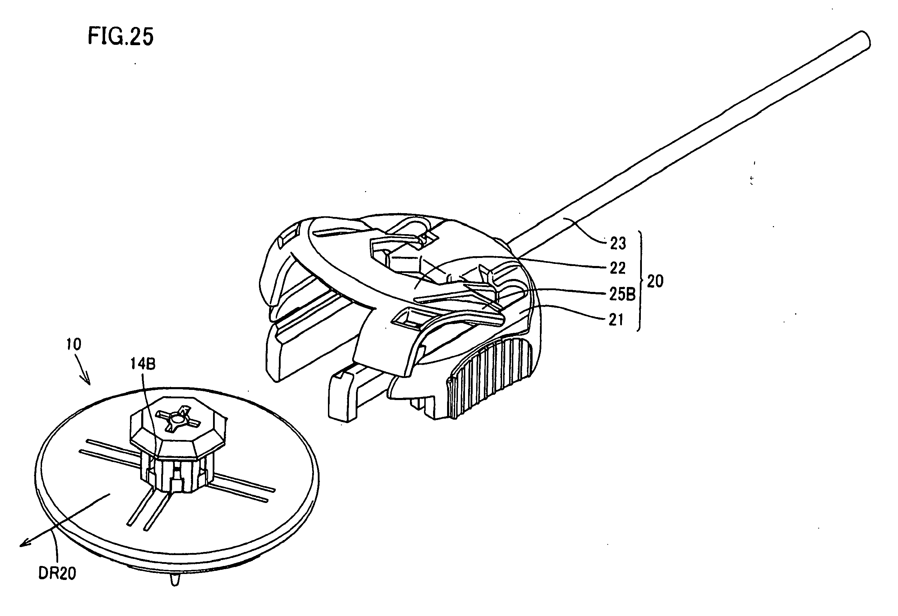

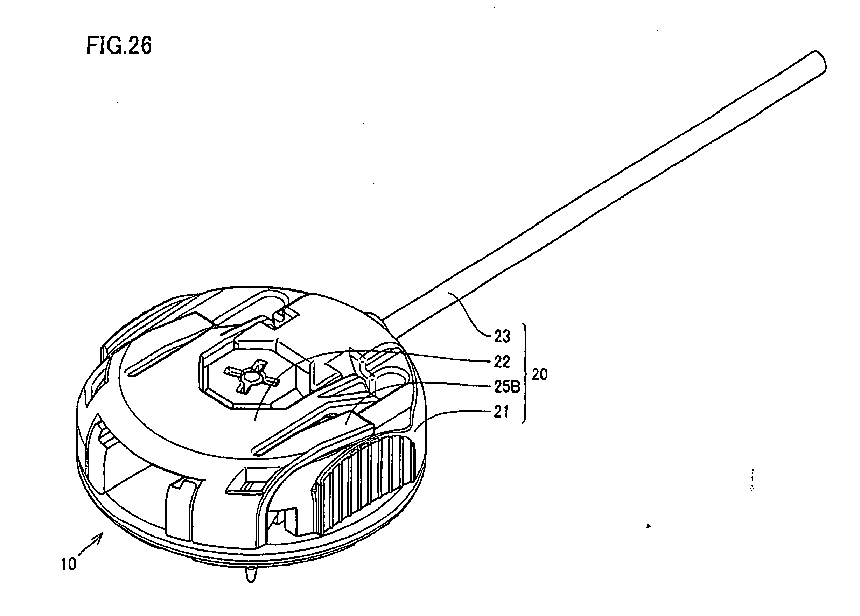

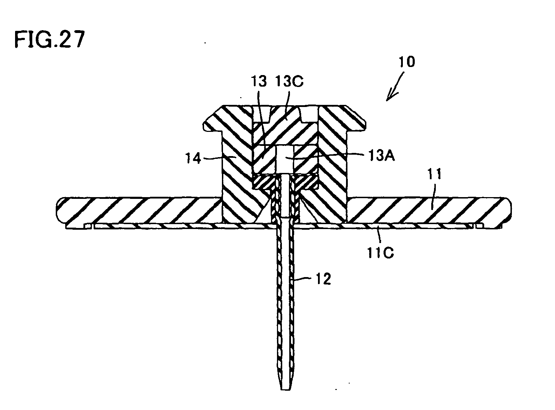

Fig. 25 is a perspective view that shows a state prior to attachment of a coupling member of a subcutaneous infusion device according to a sixth embodiment to an indwelling member. Fig. 26 is a perspective view that shows a state where the coupling member of the above-described subcutaneous infusion device is attached to the indwelling member. Further, Fig. 27 is a cross-sectional view of the indwelling member shown in Figs. 25 and 26.

With reference to Figs. 25-27, a subcutaneous infusion device according to the present embodiment is a modification of the subcutaneous infusion devices according to the first to fifth embodiments, characterized to have a feature obtained by combining the characteristic portion of the subcutaneous infusion device according to the second embodiment and the characteristic portion of the subcutaneous infusion device according to the third embodiment.

In other words, in the subcutaneous infusion device according to the present embodiment, an elastic portion 25B is provided at lid portion 22. One end side of elastic portion 25B functions as a fixed end, and the other end side of elastic portion 25B functions as a free end. As in the second embodiment, in the state where locking of main body portion 21 and lid portion 22 is released (Fig. 25), the free end of elastic portion 25B projects somewhat outward from the edge of the fitting portion of main body portion 21, and in the state where lid portion 22 is locked to main body portion 21 (Fig. 26), the free end of elastic portion 25B is deformed to be oriented somewhat inward. Therefore, when the locking of main body portion 21 and lid portion 22 is released again, lid portion 22 more easily turns in the locking-releasing direction by biasing force of elastic portion 25B.

With reference to Fig. 27, indwelling member 10 includes base portion 11, cannula 12, partition portion 13, and fastening portion 14 as in the first to fifth embodiments. Partition portion 13 is fixed to fastening portion 14 by a fixing member 13B. It is noted that Fig. 27 shows an adhesion layer 11 C provided at a bottom surface of base portion 11.

An insertion hole 14B is provided at fastening portion 14 (see Fig. 25). As in the third embodiment, needle 20B of coupling member 20 (not shown in Figs. 25-27) reaches partition portion 13 through insertion hole 14B.

In the subcutaneous infusion device according to the present embodiment, locking of lid portion 22 can easily be released as in the first to fifth embodiments.

Although the present invention has been described and illustrated in detail, it is clearly understood that the same is by way of illustration and example only and is not to be taken by way of limitation, the scope of the present invention being interpreted by the terms of the appended claims.

## Claims

1. A subcutaneous infusion device for infusing a medical solution into a body of a user, comprising:
an indwelling member (10) having
a base portion (11) fixable to a surface of the body of said user, and
a medical solution passage (12) configured to reach an inside of the body of said user when the base portion (11) is fixed to the surface of the body of the user;
a coupling member (20) attached to said indwelling member (10) in a detachable/attachable manner by being moved in a sliding manner along an extending direction of said base portion (11), wherein
said coupling member (20) includes
a main body portion (21),
a needle-like medical solution supply portion (20B) provided at said main body portion (21) and communicated with said medical solution passage (12) when said coupling member (20) is attached to said indwelling member (10),
**characterised by**
a lid portion (22) provided turnably with respect to said main body portion (21),
a lock mechanism (22A) providing locking of said lid portion (22) to said main body portion (21), and
a biasing portion (25) capable of biasing said lid portion (22) in a turning direction, when said locking is being released, by biasing force generated when said lid portion (22) is locked to said main body portion (21).

2. The subcutaneous infusion device according to claim 1, wherein said coupling member (20) has a hook portion (27) which suppresses a sliding movement of said coupling member (20) along the extending direction of said base portion (11) when said lid portion (22) is locked to said main body portion (21).

3. The subcutaneous infusion device according to claim 1 or 2, wherein
said main body portion (21) of said coupling member (20) is formed into an U shape, and
the locking of said lid portion (22) to said main body portion (21) is released by increasing a spacing between opposite ends of said U shape.

4. The subcutaneous infusion device according to any of claims 1 to 3, wherein
said indwelling member (10) has a protruding portion (13, 11 A) which protrudes outward from the body of said user with respect to said base portion (11), and
the main body portion (21) of said coupling member (20) has an opening which receives said protruding portion ( 13, 11 A).

5. The subcutaneous infusion device according to any of claims 1 to 4, wherein said indwelling member (10) has a rotation-suppressing portion which suppresses rotation of said coupling member (20) about an axis orthogonal to the extending direction of said base portion (11) when said coupling member (20) is attached to the indwelling member (10).

6. The subcutaneous infusion device according to any of claims 1 to 5, wherein
said indwelling member (10) has a flange portion (14A),
said coupling member (20) has a rail portion (26) which engages with said flange portion (14A), and
said flange portion (14A) and said rail portion (26) suppress a movement of said coupling member (20) along a direction orthogonal to the extending direction of said base portion (11) when said coupling member (20) is attached to said Indwelling member (10).

7. The subcutaneous infusion device according to any of claims 1 to 6, wherein said coupling member (20) includes a cover member (20A) fixed to said main body portion (21), formed to cover said medical solution supply portion (20B) when said coupling member (20) is detached from said indwelling member (10), and formed to receive compression force from said indwelling member (10) and be compressed in an axial direction to thereby allow said medical solution supply portion (20B) to penetrate the cover member (20A) and allow the medical solution supply portion (20B) and said medical solution passage (12) to be communicated with each other, when said coupling member (20) is attached to said indwelling member (10).

8. The subcutaneous infusion device according to claim 7, wherein
said indwelling member (10) has a protruding portion (13, 11A) which protrudes outward from the body of said user with respect to said base portion (11), and
said cover member (20A) abuts on said protruding portion (13, 11 A) when said coupling member (20) is attached to said indwelling member (10).

9. The subcutaneous infusion device according to claim 7 or 8, wherein said cover member (20A) has a self-closing property which enables a hole portion formed by penetration of said medical solution supply portion (20B) to be closed when said coupling member (20) is detached from said indwelling member (10).

10. The subcutaneous infusion device according to any of claims 7 to 9, wherein a concave portion (200A) is provided at a side surface of said cover member (20A).

11. The subcutaneous infusion device according to any of claims 1 to 10, wherein
said indwelling member (10) further has a partition portion (13) which defines said medical solution passage (12) and a fastening portion (14) which fastens the partition portion (13) onto said base portion,
said partition portion (13) includes a puncture portion (13) into which said medical solution supply portion (20B) is inserted, and
the puncture portion (13) is formed to protrude more upward than said fastening portion (14) protrudes in a state where said medical solution supply portion (20B) is detached from said indwelling member.

12. The subcutaneous infusion device according to claim 11, wherein said coupling member (20) further includes a press portion (28) which presses said partition portion (13) when the coupling member (20) is attached to said indwelling member (10).

13. The subcutaneous infusion device according to claim 11 or 12, further comprising a cover member (20A) fixed to said coupling member (20) and formed to cover said medical solution supply portion (20B) when said coupling member (20) is detached from said indwelling member (10), wherein
said cover member (20A) is formed to abut on said indwelling member (10) and receive compression force to thereby be compressed in an axial direction, and allow said medical solution supply portion (20B) to penetrate said cover member (20A) and said puncture portion (13) and allow the medical solution supply portion (28B) to be communicated with said medical solution passage (12), when said coupling member (20) is attached to said indwelling member (10).

14. The subcutaneous infusion device according to any of claims 11 to 13, wherein said puncture portion (13) of said indwelling member (10) has a self-closing property which enables a hole portion formed by penetration of said medical solution supply portion (12) to be closed when said coupling member (20) is detached from said indwelling member (10).

## Patentansprüche

1. Subkutane Infusionsvorrichtung zum Infundieren einer medizinischen Lösung in einen Körper eines Benutzers, umfassend:
ein Einsatzteil (10) mit
einem Basisabschnitt (11), welcher an einer Oberfläche des Körpers des Benutzers befestigbar ist, und
einem Kanal (12) für die medizinische Lösung, welcher derart ausgestaltet ist, dass er ein Inneres des Körpers des Benutzers erreicht, wenn der Basisabschnitt (11) an der Oberfläche des Körpers des Benutzers befestigt ist;
ein Kopplungsteil (20), welches an dem Einsatzteil (10) in einer lösbaren/anbringbaren Weise angebracht ist, indem es in einer gleitenden Weise entlang einer Verlaufsrichtung des Basisabschnitts (11) bewegt wird,
wobei das Kopplungsteil (20) umfasst
einen Hauptkörperabschnitt (21),
einen nadelähnlichen Zuführungsabschnitt (20B) für die medizinische Lösung,
welcher an dem Hauptkörperabschnitt (21) vorhanden ist und mit dem Kanal (12) für die medizinische Lösung kommuniziert, wenn das Kopplungsteil (20) an
dem Einsatzteil (10) angebracht ist,
**gekennzeichnet durch**
einen Deckelabschnitt (22), welcher biegbar bezüglich des Hauptkörperabschnitts (21) vorhanden ist,
einen Verriegelungsmechanismus (22A), welcher eine Verriegelung des Deckelabschnitts (22) an dem Hauptkörperabschnitt (21) ermöglicht, und
einen Vorspannabschnitt (25), welcher in der Lage ist, den Deckelabschnitt (22) in einer gekrümmten Richtung vorzuspannen, wenn die Verriegelung gelöst wird, indem die Vorspannkraft erzeugt wird, wenn der Deckelabschnitt (22) mit dem Hauptkörperabschnitt (21) verriegelt wird.

2. Subkutane Infusionsvorrichtung nach Anspruch 1,
**dadurch gekennzeichnet,**
**dass** das Kopplungsteil (20) einen Hakenabschnitt (27) aufweist, welcher eine gleitende Bewegung des Kopplungsteils (20) entlang der Verlaufrichtung des Basisabschnitts (11) verhindert, wenn der Deckelabschnitt (22) mit dem Hauptkörperabschnitt (21) verriegelt ist.

3. Subkutane Infusionsvorrichtung nach Anspruch 1 oder 2,
**dadurch gekennzeichnet,**
**dass** der Hauptkörperabschnitt (21) des Kopplungsteils (20) in einer U-Form ausgebildet ist, und
**dass** das Verriegeln des Deckelabschnitts (22) mit dem Hauptkörperabschnitt (21) gelöst wird, indem ein Abstand zwischen gegenüberliegenden Enden der U-Form vergrößert wird.

4. Subkutane Infusionsvorrichtung nach einem der Ansprüche 1 bis 3,
**dadurch gekennzeichnet,**
**dass** das Einsatzteil (10) einen hervorragenden Abschnitt (13, 11A) aufweist, welcher von dem Körper des Benutzers bezüglich des Basisabschnitts (11) nach außen hervorragt, und
**dass** der Hauptkörperabschnitt (21) des Kopplungsteils (20) eine Öffnung aufweist, welche den hervorragenden Abschnitt (13, 11A) aufnimmt.

5. Subkutane Infusionseinrichtung nach einem der Ansprüche 1 bis 4,
**dadurch gekennzeichnet,**
**dass** das Einsatzteil (10) einen Drehunterdrückungsabschnitt aufweist, welcher eine Drehung des Kopplungsteils (20) um eine Achse orthogonal zu der Verlaufsrichtung des Basisabschnitts (11) unterdrückt, wenn das Kopplungsteil (20) an dem Einsatzteil (10) angebracht ist.

6. Subkutane Infusionseinrichtung nach einem der Ansprüche 1 bis 5,
**dadurch gekennzeichnet,**
**dass** das Einsatzteil (10) einen Flanschabschnitt (14A) aufweist,
**dass** das Kopplungsteil (20) einen Schienenabschnitt (26) aufweist, welcher sich mit dem Flanschabschnitt (14A) in Eingriff befindet, und
**dass** der Flanschabschnitt (14A) und der Schienenabschnitt (26) eine Bewegung des Kopplungsteils (20) entlang einer Richtung orthogonal zu der Verlaufsrichtung des Basisabschnitts (11) verhindern, wenn das Kopplungsteil (20) an dem Einsatzteil (10) angebracht ist.

7. Subkutane Infusionseinrichtung nach einem der Ansprüche 1 bis 6,
**dadurch gekennzeichnet,**
**dass** das Kopplungsteil (20) ein Abdeckteil (20A) umfasst, welches an dem Hauptkörperabschnitt (21) befestigt ist, welches derart ausgestaltet ist, dass es den Zuführungsabschnitt (20B) für die medizinische Lösung abdeckt, wenn das Kopplungsteil (20) von dem Einsatzteil (10) gelöst wird, und welches derart ausgestaltet ist, dass es eine Kompressionskraft von dem Einsatzteil (10) aufnimmt und in eine axialen Richtung zusammengedrückt wird, um **dadurch** dem Zuführungsabschnitt (20B) für die medizinische Lösung zu ermöglichen, dass Abdeckteil (20A) zu durchdringen und um dem Zuführungsabschnitt (20B) für die medizinische Lösung und dem Kanal (12) für die medizinische Lösung zu ermöglichen, miteinander zu kommunizieren, wenn das Kopplungsteil (20) an dem Einsatzteil (10) angebracht ist.

8. Subkutane Infusionseinrichtung nach Anspruch 7,
**dadurch gekennzeichnet,**
**dass** das Einsatzteil (10) einen hervorragenden Abschnitt (13, 11A) aufweist, welcher von dem Körper des Benutzers bezüglich des Basisabschnitts (11) nach außen hervorragt, und
**dass** das Abdeckteil (20A) gegen den hervorragenden Abschnitt (13, 11A) stößt, wenn das Kopplungsteil (20) an dem Einsatzteil (10) angebracht ist.

9. Subkutane Infusionseinrichtung nach Anspruch 7 oder 8,
**dadurch gekennzeichnet,**
**dass** das Abdeckteil (20A) eine selbst verschließende Eigenschaft aufweist, welche einem Lochabschnitt, welcher durch eine Durchdringung des Zuführungsabschnitts (20B) für die medizinische Lösung ausgebildet wird, geschlossen wird, wenn das Kopplungsteil (20) von dem Einsatzteil (10) gelöst wird.

10. Subkutane Infusionsvorrichtung nach einem der Ansprüche 7 bis 9,
**dadurch gekennzeichnet,**
**dass** ein konkaver Abschnitt (200A) an einer Seitenoberfläche des Abdeckteils (20A) vorhanden ist.

11. Subkutane Infusionsvorrichtung nach einem der Ansprüche 1 bis 10,
**dadurch gekennzeichnet,**
**dass** das Einsatzteil (10) darüber hinaus einen Teilungsabschnitt (13) aufweist, welcher den Kanal (12) für die medizinische Lösung und einen Befestigungsabschnitt (14) definiert, welcher den Teilungsabschnitt (13) auf dem Basisabschnitt befestigt,
**dass** der Teilungsabschnitt (13) einen Punktionsabschnitt (13) aufweist, in welchen der Zuführungsabschnitt (20B) für die medizinische Lösung eingeführt ist, und
**dass** der Punktionsabschnitt (13) derart ausgebildet ist, dass er in einem Zustand mehr als der Befestigungsabschnitt (14) nach oben hervorragt, in welchem der Zuführungsabschnitt (20B) für die medizinische Lösung von dem Einsatzteil gelöst ist.

12. Subkutane Infusionsvorrichtung nach Anspruch 11,
**dadurch gekennzeichnet,**
**dass** das Kopplungsteil (20) darüber hinaus einen Druckabschnitt (28) aufweist, welcher den Teilungsabschnitt (13) drückt, wenn das Kopplungsteil (20) an dem Einsatzteil (10) angebracht ist.

13. Subkutane Infusionsvorrichtung nach Anspruch 11 oder 12,
**dadurch gekennzeichnet,**
**dass** die Infusionsvorrichtung darüber hinaus ein Abdeckteil (20A) umfasst, welches an dem Kopplungsteil (20) befestigt ist und welches derart ausgestaltet ist, dass es den Zuführungsabschnitt (20B) für die medizinische Lösung abdeckt, wenn das Kopplungsteil (20) von dem Einsatzteil (10) gelöst wird,
wobei das Abdeckteil (20A) derart ausgestaltet ist, dass es gegen das Einsatzteil (10) stößt und eine Kompressionskraft aufnimmt, um **dadurch** in einer axialen Richtung zusammengedrückt zu werden und dem Zuführungsabschnitt (20B) für die medizinische Lösung ermöglicht, dass Abdeckteil (20A) und den Punktionsabschnitt (13) zu durchdringen und dem Zuführungsabschnitt (28B) für die medizinische Lösung ermöglicht, mit dem Kanal (12) für die medizinische Lösung zu kommunizieren, wenn das Kopplungsteil (20) an dem Einsatzteil (10) angebracht ist.

14. Subkutane Infusionsvorrichtung nach einem der Ansprüche 11 bis 13,
**dadurch gekennzeichnet,**
**dass** der Punktionsabschnitt (13) des Einsatzteils (10) eine selbst verschließende Eigenschaft aufweist, welche ermöglicht, dass ein Lochabschnitt, welcher durch Durchdringen des Zuführungsabschnitts (12) der medizinischen Lösung ausgebildet ist, verschlossen wird, wenn das Kopplungsteil (20) von dem Einsatzteil (10) gelöst wird.

## Revendications

1. Dispositif de perfusion sous-cutanée pour perfuser une solution médicale dans un corps d'un utilisateur, comprenant :
un élément à demeure (10) ayant :
une partie de base (11) pouvant être fixée sur une surface du corps dudit utilisateur, et
un passage de solution médicale (12) configuré pour atteindre un intérieur du corps dudit patient lorsque la partie de base (11) est fixée sur la surface du corps de l'utilisateur ;
un élément de couplage (20) fixé sur ledit élément à demeure (10) d'une manière détachable/fixable en étant déplacé d'une manière coulissante le long d'une direction d'extension de ladite partie de base (11), dans lequel :
ledit élément de couplage (20) comprend :
une partie de corps principal (21),
une partie d'alimentation de solution médicale en forme d'aiguille (20B) prévue au niveau de ladite partie de corps principal (21) et communiquant avec ledit passage de solution médicale (12) lorsque ledit élément de couplage (20) est fixé audit élément à demeure (10),
**caractérisé par** :
une partie de couvercle (22) prévue de manière rotative par rapport à ladite partie de corps principal (21),
un mécanisme de blocage (22A) fournissant le blocage de ladite partie de couvercle (22) sur ladite partie de corps principal (21), et
une partie de sollicitation (25) pouvant solliciter ladite partie de couvercle (22) dans une direction rotative, lorsque ledit blocage est débloqué, par la force de sollicitation générée lorsque ladite partie de couvercle (22) est bloquée sur ladite partie de corps principal (21).

2. Dispositif de perfusion sous-cutanée selon la revendication 1, dans lequel ledit élément de couplage (20) a une partie de crochet (27) qui supprime un mouvement coulissant dudit élément de couplage (20) le long de la direction d'extension de ladite partie de base (11) lorsque ladite partie de couvercle (22) est bloquée sur ladite partie de corps principal (21).

3. Dispositif de perfusion sous-cutanée selon la revendication 1 ou 2, dans lequel :
ladite partie de corps principal (21) dudit élément de couplage (20) est formée selon une forme de U, et
le blocage de ladite partie de couvercle (22) sur ladite partie de corps principal (21) est débloqué en augmentant un espacement entre les extrémités opposées de ladite forme de U.

4. Dispositif de perfusion sous-cutanée selon l'une quelconque des revendications 1 à 3, dans lequel :
ledit élément à demeure (10) a une partie en saillie (13, 11A) qui fait saillie vers l'extérieur à partir du corps dudit utilisateur par rapport à ladite partie de base (11), et
la partie de corps principal (21) dudit élément de couplage (20) a une ouverture qui reçoit ladite partie en saillie (13, 11A).

5. Dispositif de perfusion sous-cutanée selon l'une quelconque des revendications 1 à 4, dans lequel ledit élément à demeure (10) a une partie de suppression de rotation qui supprime la rotation dudit élément de couplage (20) autour d'un axe orthogonal à la direction d'extension de ladite partie de base (11) lorsque ledit élément de couplage (20) est fixé sur l'élément à demeure (10).

6. Dispositif de perfusion sous-cutanée selon l'une quelconque des revendications 1 à 5, dans lequel :
ledit élément à demeure (10) a une partie de rebord (14A),
ledit élément de couplage (20) a une partie de rail (26) qui se met en prise avec ladite partie de rebord (14A), et
ladite partie de rebord (14A) et ladite partie de rail (26) suppriment un mouvement dudit élément de couplage (20) le long d'une direction orthogonale à la direction d'extension de ladite partie de base (11) lorsque ledit élément de couplage (20) est fixé audit élément à demeure (10).

7. Dispositif de perfusion sous-cutanée selon l'une quelconque des revendications 1 à 6, dans lequel ledit élément de couplage (20) comprend un élément de couvercle (20A) fixé sur ladite partie de corps principal (21), formé afin de recouvrir ladite partie d'alimentation de solution médicale (20B) lorsque ledit élément de couplage (20) est détaché dudit élément à demeure (10), et formé pour recevoir la force de compression provenant dudit élément à demeure (10) et être comprimé dans une direction axiale pour permettre ainsi à ladite partie d'alimentation de solution médicale (20B) de pénétrer dans l'élément de couvercle (20A) et permettre à la partie d'alimentation de solution médicale (20B) et audit passage de solution médicale (12) de communiquer entre eux, lorsque ledit élément de couplage (20) est fixé audit élément à demeure (10).

8. Dispositif de perfusion sous-cutanée selon la revendication 7, dans lequel :
ledit élément à demeure (10) a une partie en saillie (13, 11A) qui fait saillie vers l'extérieur à partir du corps dudit utilisateur par rapport à ladite partie de base (11), et
ledit élément de couvercle (20A) vient en butée sur ladite partie en saillie (13, 11A) lorsque ledit élément de couplage (20) est fixé audit élément à demeure (10).

9. Dispositif de perfusion sous-cutanée selon la revendication 7 ou 8, dans lequel ledit élément de couvercle (20A) a une propriété de fermeture automatique qui permet à une partie de trou formée par la pénétration de ladite partie d'alimentation de solution médicale (20B) d'être fermée lorsque ledit élément de couplage (20) est détaché dudit élément à demeure (10).

10. Dispositif de perfusion sous-cutanée selon l'une quelconque des revendications 7 à 9, dans lequel une partie concave (200A) est prévue au niveau d'une surface latérale dudit élément de couvercle (20A).

11. Dispositif de perfusion sous-cutanée selon l'une quelconque des revendications 1 à 10, dans lequel :
ledit élément à demeure (10) a en outre une partie de séparation (13) qui définit ledit passage de solution médicale (12) et une partie de fixation (14) qui fixe la partie de séparation (13) sur ladite partie de base,
ladite partie de séparation (13) comprend une partie de perforation (13) dans laquelle ladite partie d'alimentation de solution médicale (20B) est insérée, et
la partie de perforation (13) est formée pour faire saillie davantage vers le haut que ladite partie de fixation (14) ne fait saillie dans un état dans lequel ladite partie d'alimentation de solution médicale (20B) est détachée dudit élément à demeure.

12. Dispositif de perfusion sous-cutanée selon la revendication 11, dans lequel ledit élément de couplage (20) comprend en outre une partie de pression (28) qui appuie sur ladite partie de séparation (13) lorsque l'élément de couplage (20) est fixé sur ledit élément à demeure (10).

13. Dispositif de perfusion sous-cutanée selon la revendication 11 ou 12, comprenant en outre un élément de couvercle (20A) fixé sur ledit élément de couplage (20) et formé pour recouvrir ladite partie d'alimentation de solution médicale (20B) lorsque ledit élément de couplage (20) est détaché dudit élément à demeure (10), dans lequel :
ledit élément de couvercle (20A) est formé pour venir en butée sur ledit élément à demeure (10) et recevoir la force de compression pour être ainsi comprimé dans la direction axiale, et permettre à ladite partie d'alimentation de solution médicale (20B) de pénétrer dans ledit élément de couvercle (20A) et ladite partie de perforation (13) et permettre à la partie d'alimentation de solution médicale (28B) de communiquer avec ledit passage de solution médicale (12) lorsque ledit élément de couplage (20) est fixé dans ledit élément à demeure (10).

14. Dispositif de perfusion sous-cutanée selon l'une quelconque des revendications 11 à 13, dans lequel ladite partie de perforation (13) dudit élément à demeure (10) a une propriété de fermeture automatique qui permet à une partie de trou formée par pénétration de ladite partie d'alimentation de solution médicale (12) d'être fermée lorsque ledit élément de couplage (20) est détaché dudit élément à demeure (10).
